# EUROPEAN PATENT APPLICATION

(11) **EP 3 141 606 A1**
(43) Date of publication of application: **15.03.2017**
(21) Application number: 16187956.4
(22) Date of filing: 09.09.2016
(51) Int. Cl.: C12P 1/02, C12P 7/04, C12P 7/56, A23K 10/14

(54) **BIOCATALYST FOR SILAGES FOR LIVESTOCK FEEDING**

(30) Priority: 10.09.2015 IT UB20153541
(71) Applicant: Methodo Chemicals S.r.l., 42017 Novellara (IT)
(72) Inventor: CERCHIARI, Emilio, 8055 GRAZ (AT); LEONARDI, Giuliano, 26031 ISOLA DOVARESE (IT)
(74) Representative: Zaccaro, Elisabetta

(57) **Abstract**

A process for the preparation of a biocatalyst of plant biomass present in the silage for livestock feeding. The process comprises mixing flour hydrolysate with one or more sugars, minerals, aminoacids, propolis and vitamins and warer, fermenting the mixture with Saccharomyces cerevisiae and pasteurizing the fermentation broth.

The biocatalyst that can be obtained by the process is used for promoting lactic fermentation, and can be used as an additive for animal and livestock food.

## Description

### FIELD OF THE INVENTION

The invention relates to a process for the preparation of a biocatalyst of the plant biomass present in the silage for livestock feeding. Also described is a biocatalyst that can be obtained by the process according to the invention promoting lactic fermentation, and its uses as an additive for animal and livestock food.

### STATE OF THE ART

The use of ensiled forage is a very common practice for feeding animals bred worldwide. Most represented forages include corn, alfalfa, rye grass and others. In addition to these, also whole grains, such as corn, wheat and oilseed extraction derivatives such as rapeseed, sunflower, soybean, are becoming more and more used, with ever increasing amounts.

The ensilage gives significant advantages to the farmer, because it allows the storage of large volumes of fodder and cereals harvested with high humidity. Therefore, fodder and cereals do not require any drying costs and can be purchased at harvest, i.e. at the market lowest price. In most cases the fermentation that can preserve the plant biomass in a natural way is the lactic type. The greatest difficulty is to start fermentation, which must take place in the quickest possible way to remove the antagonism of other microorganisms that facilitate putrefaction, or yeast.

The limiting and unfavorable factor for lactic fermentation is the food buffer capacity that tends to maintain high pH levels in the biomass. High pH levels can advance growth of putrefactive microorganisms, yeasts and molds. It is in fact a fermentation that depends on the presence of different types of microorganisms, which are naturally present in the biomass. External factors, such as nutritional additives or microorganisms, can be introduced to facilitate the lactic fermentation. The preservation of forage is based on the removal of oxygen from the mass and the rapid increase in acidity of the same, through lactic microbial fermentation. A lactic fermentation takes place through different phases which are continuous. Schematically:
1. Aerobic phase: The biomass introduced into the fermenter continues for some time to breathe. Plant cells use oxygen and degrade the sugars, whose combustion creates heat. At the same time the native flora begins to degrade/brew and the biomass helps to eliminate residual oxygen that is converted into carbon dioxide, useful for the control on the development of molds. Oxygen removal needs to be very fast because the biomass cells use it and cause the temperature to increase up to levels which can reduce the nutritional value of biomass.
2. Lag phase: the plant cell walls are broken and spill out the cell sap that becomes food for microbial flora. In this way the growth of microorganisms is increased. In this initial bacterial growing phase, the number of cells remains relatively constant before a subsequent rapid increase. This is a slow process that can become very critical.
3. Fermentation phase: The lactic flora - that produces lactic acid - begins to dominate the fermentation process after the pH reaches values of 5.5 - 5.7, as compared to the initial ones at ensilage (6.5 - 6.7). The pH reduction rate depends greatly on the previous phase (2). Actually, phase 2 and phase 3 proceed with continuity. Adequate lactic acid production requires that certain basic parameters are met:
   a- Values of pH in the range of 5 to 5.5
   b- Availability of sugar
   c- High presence of lactic acid bacteria
   d- Anaerobic conditions
   Once the above parameters are satisfied, also a proper management of the various stages of collection and ensilage is necessary to ensure anaerobic conditions and a sufficient level of humidity. With a proper ensilage process, the lactic acid production lowers the pH values of the plant biomass at 4.4 - 5.0 in fodders with high buffering capacity and low carbohydrate availability (for example legumes), in cereals (corn, sorghum etc.) and oat grass, that has rather low buffering capacity and a greater amount of available carbohydrates, allow obtainment of silages with a final pH of 3.8 - 4.2.
4. Stability phase: the lowering of pH values blocks the bacterial fermentation and the silage stabilizes. If the presence of lactic acid is not sufficient, some bacteria, such as Clostridia, may produce butyric acid and damage the protein of the biomass.

Different products are known and used as additives for the preservation of forage, the purpose of these products being to facilitate a rapid development of lactic acid bacteria.

The products used so far are bacterial inoculants, sugars and enzymes:
Bacterial inoculants: the living microorganisms present in the inoculants must meet certain requirements, e.g., they must be able to grow quickly in a wide range of conditions and take advantage over other less desirable bacteria; they must produce only lactic acid exploiting the fermentation of glucose, fructose and
sucrose; Finally, they must be acid-tolerant and ensure a final pH sufficiently low for the biomass in fermentation. The microorganisms used are different species of lactobacilli.

Sugars: substances such as molasses, glucose and dextrose can be added to the biomass to be fermented, so as to provide a selective fermentation medium to the native lactobacilli. The addition of sugars helps to reduce losses in the dry matter and improves the stability.

Enzyme substrates: they are used to break down the complex sugars found in plant biomass. The most common sources, e.g. *Aspergillus oryzae, Aspergillus niger* and *Bacillus subtilis,* contain enzymes - cellulase, hemicellulase and amylase - indeed capable of acting on cellulose, hemicellulose and starch providing a suitable substrate to lactic flora.

The products used up to now based on bacterial inocula, sugars and enzymes have the following disadvantages:
a- Sugars: they are difficult to dose because they have to be assessed on the basis of the type of fodder to be ensiled. An excess may give rise to bad fermentation and especially to an increase in temperature of the biomass which causes excessive loss of nutrients. The sugars are foremost used by the fodder cells.
b- Bacterial inoculum: this is typically a Lactobacillus plantarum with different strains of Streptococcus and Pediococcus. Their survival within the biomass may be critical, depending on the initial pH values and temperatures that develop in the ensiled biomass. Their dosage is very difficult due to the great variety of fodders for silage and for the heavy seasonal influence on crops. Mixing with the biomass for silage must be very accurate and many times this is hampered by the state of the art present in farms. In addition to this, their quantity must be much higher than the natural presence of lactobacilli in the biomass, in order to be functional. Finally, they must have a large amount of sugar available to start fermentation.
c- Enzyme substrates: they are used to break down the complex sugars present in the fodder, to obtain other more simple and better used by lactic acid bacteria. The most common sources, e.g. Aspergillus oryzae, Aspergillus niger and Bacillus subtilis, contain enzymes - cellulase, hemicellulase and amylase - indeed capable of acting on cellulose, hemicellulose and starch. The enzyme substrates are active if plant sugars are very low and in the presence of a large amount of lactic acid bacteria. These are conditions quite difficult to find, so very high doses are applied and associated with specific bacterial cultures (lactobacilli), in order to obtain a minimum of activity. These additives are particularly effective for fodder with a high moisture content (60 - 70%) and with the possibility of prolonged ensiling times. The silage should be allowed to ferment for at least three months. Moreover, enzymes are thermo-labile, therefore, in many situations they are neutralized in the initial phase of silage.

They are also not recommended for ensilage of corn crop, because of their high content of sugar that can give rise to yeasts and alcohol production. Corn is the most widely used crop for silage in many parts of the world, so this factor is very penalizing.

To sum up, the action of known additives is primarily a direct action, and as such it runs out very quickly. Also, this feature requires an individual study of material to be ensiled, theoretically batch by batch. Ideal conditions for their correct use are not easily found. Furthermore, in many occasions the condition appears to be antagonists (for example enzymes vs. sugars).

Object of the present invention is therefore to provide a nutritional additive to plant biomass that characterizes the silage for livestock use, which promotes lactic fermentation, lowers pH values, hampers the growth of putrefactive microorganisms, yeasts and molds, and that do not has the disadvantages of known products in the same field, as described above.

### SUMMARY OF THE INVENTION

Therefore, the invention relates to a process for the preparation of a biocatalyst comprising the steps of:
a. mixing an amount of flour hydrolyzate with one or more sugars, minerals, aminoacids, propolis and vitamins thereby producing a dry mixture;
b. adding water to the dry mixture of step a. in order to obtain a suspension;
c. fermenting the suspension of step b. with yeast belonging to the Saccharomyces cerevisiae species for about 10-14 hours at a temperature in the range of 25°C to 35°C, preferably at 31 °C, in order to obtain a fermentation broth; and
d. pasteurizing the fermentation broth of step c. at a temperature in the range of 85°C to 90°C for about 15-20 minutes.

Also described is a biocatalyst that can be obtained by the process according to the present invention.

In a further embodiment, the present invention relates to the use of the biocatalyst as an additive for animal and livestock food.

### DESCRIPTION OF FIGURES

The invention will now be described in detail and with reference to the accompanying Figures in which:
Figure 1a, shows a graph plotting a classical fermentation of a plant biomass ensiled for livestock use; and
Figure 1b, shows a graph plotting the trend of a classical fermentation of a plant biomass ensiled for livestock use with the addition of a biocatalyst according to the invention.

The data are described in Example 2.

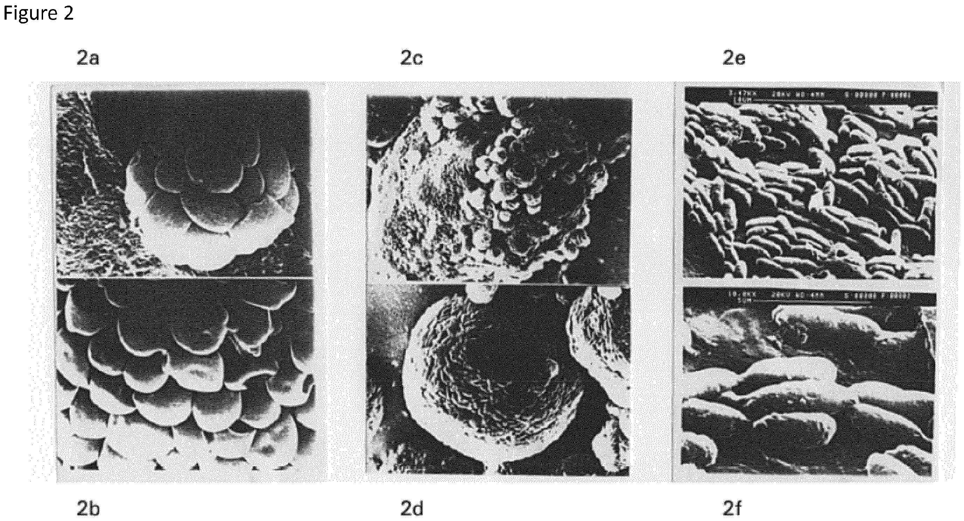

Figure 2: electron microscope image of a Rapsody granule.
Figure 2a shows the aggregated S. *cerevisiae* intact cells (8,470x magnification).
Figure 2b represents S. *cerevisiae* intact cells arranged thoroughly with an initial rupture of the membranes (10,000x magnification).
Figure 2c represents S. *cerevisiae* cells arranged on a complex organic-mineral structure (3,470x magnification).
Figure 2d shows a granule is covered by S. *cerevisiae* intact cells and their aggregated cell membranes (10,000x magnification).
Figure 2e represents S. *cerevisiae* cell walls associated with organic-mineral granules of a different nature (3,470x magnification).
Figure 2f shows complex granules of phospho-carbo-pepto chelation complexes aggregated to S. *cerevisiae* cell walls (10,000x magnification).

### DETAILED DESCRIPTION OF THE INVENTION

The invention thus relates to a biocatalyst for the fermentation of plant biomass intended for livestock feeding, with selective activity on native lactic acid bacteria. Unlike the products already known in this field, the biocatalyst in accordance with the present invention does not add live microorganisms but only metabolites and products of fermentation with S. *cerevisiae,* from selected substrates.

The purpose of the product according to the invention is to facilitate a rapid development of lactic acid bacteria, therefore a specific preparation and suitable for stimulation of fermentative capacity of native lactobacilli in ensiled plant biomass for livestock feeding, obtained by fermentation of *Saccharomyces cerevisiae.*

Therefore, the invention relates to a process for the preparation of a biocatalyst comprising the steps of:
a. mixing an amount of flour hydrolyzate with one or more sugars, minerals, aminoacids, propolis and vitamins thereby producing a dry mixture;
b. adding water to the dry mixture of step a. in order to obtain a suspension;
c. fermenting the suspension of step b. with yeast belonging to the *Saccharomyces cerevisiae* species for about 10-14 hours at a temperature in the range of 25°C to 35°C, preferably at a temperature of 31 °C, in order to obtain a fermentation broth;
d. pasteurizing the fermentation broth of step c. at a temperature in the range of 85°C to 90°C for about 15-20 minutes.

The use of propolis has proven to be the most suitable for its natural antiseptic properties. Substitution of the propolis can only take place with an antibiotic with all the negative aspects associated with its use. Vitamins are essential as growth factors of useful microorganisms. Fermentation could take place even without the use of vitamins but it would be slower and less efficient.

In the present invention when using the term:
- "biocatalyst", a product that is obtained by fermentation of selected substrates with *Saccharoyces cerevisiae* is meant, which is based on mutual symbiosis between native microorganisms, and is able to enhance specific functions.

The integral derivative of fermentation before specifically usinf it as a biocatalyst is pasteurized to avoid contamination and/or unusual modulations in the biomass to be fermented. Alive microorganisms can in fact create competitive phenomena, and many times even negative ones, to the detriment of microorganisms naturally present in the biomass. In addition to this, to have a selective activity on native lactic acid bacteria, they should be inoculated in an amount 10 times higher than those naturally present in the biomass.

In a preferred embodiment, in the process according to the present invention, the flour hydrolyzate is preferably a flour hydrolyzate of soy, peas, rape, sunflowers, yeast auto-lysate, wheat by-products, potato protein concentrate or a mixture thereof.

In a further embodiment, in the process according to the present invention, the sugar is selected from the group consisting of dextrose, glycerol, mannose, sucrose, fructose or a mixture thereof.

Advantageously, in the process according to the present invention, minerals are chosen from the group consisting of phosphates, sulphates, mineral microelements, preferably mono-ammonic phosphate, bicalcic orthophosphate, monosodium phosphate, sodium sulphate, zinc chloride, zinc sulphate, zinc oxide, copper sulphate, copper chloride, iron sulphate, iron chloride, manganese sulphate, sulphur flower or a mixture thereof.

In one embodiment, in the process according to the present invention, the amino acids are chosen from the group consisting of lysine, methionine, threonine, tryptophan, valine, cysteine, arginine or a mixture thereof.

In a preferred embodiment, in the process according to the present invention, the dry mixture of step a. comprises soy hydrolysate, food sugar, (sucrose, glucose), glicerol, mono-ammonic phosphate, bicalcic orthophosphate, monosodium phosphate, sodium sulphate, lysine, methionine, zinc chloride, copper sulphate, iron sulphate, manganese sulphate.

In a most preferred embodiment, in the process according to the present invention, the dry mixture of step a. comprises soy hydrolysate, food sugar, (sucrose, glucose), glicerol, mono-ammonic phosphate, bicalcic orthophosphate, monosodium phosphate, sodium sulphate, lysine, methionine, zinc chloride, copper sulphate, iron sulphate, manganese sulphate, in the following quantities for liter of water, preferably deionised water:
Also described is a biocatalyst that can be obtained by the process according to the present invention.

The biocatalyst according to the present invention has the following advantages, as compared to the known products for the same application: It is active on any substrate, as it acts indirectly as a specific and selective nourishment of native lactobacilli. The product infact allows the promotion of lactic fermentation, as it lowers the pH values inside the silo, hampers the growth of putrefactive microorganisms, yeasts and molds that would damage the crop.

### Action model of the biocatalyst:

The biocatalyst according to the present invention is called "RAPSODY", and is a preparation obtained by specific phosphorylation techniques that drive the native lactic fermentation of plant biomass during ensilation. The organic transformation of elements and natural raw materials operated by the *Saccharomyces cerevisiae* in the long fermentation phase in association with complex phosphorylation processes, provides unique growth material for the lactic acid microorganisms to any level of pH and temperature. The biocatalyst does not add living microorganisms, so it do not unbalance the biomass fermentation processes. The biocatalyst has a booster action on the growth speed of lactic acid bacteria and makes them competitive and rapidly dominant over the rest of the native micro-population. The biocatalyst starts its action by stimulating the growth of the active Lactobacilli even at high pH values of 6-6.5. These lactic acid bacteria, in turn, due to their metabolism, induce rapid lowering of the pH to values of 5 to 5.3 in the first 12-24 hours. The growth of active lactic acid bacteria, such as *Lactobacillus plantarum,* is thus selected and exalted to these values. *L. plantarum* is the most important microorganism necessary for the success of a high quality fermentation in ensiled plant biomass.

The main activity of RAPSODY is to provide nutrients that can accelerate the growth of lactobacilli avoiding heavy losses of nutritional energy to detriment of the sugars due to cell respiration phenomena of plant biomass. This avoids the risk of overheating of the biomass and at the same time leads to a rapid acidification due to the native lactobacilli that ferment the organic substance up to the production of organic acids. RAPSODY directly promotes the metabolism of microorganisms and stimulates the development of lactobacilli, in particular *L. plantarum.*

In a further embodiment, the present invention relates to the use of the biocatalyst as an additive for animal and livestock food.

An example of such animal and livestock food, is a silage.

Said animal and livestock food is in a solid or liquid form or has a water percentage of 15% to 80%, preferably 20% to 80%.

Experimental data have shown that lowering of the pH-value is associated with an increase in lactic acid concentrations. In basal conditions (pH around 7 or low glucose content) concentrations of propionic acid and acetic acid are relatively high as compared to those of lactic acid. When high concentrations of fermentable carbohydrates occur, the production of lactic acid increases drastically. The nature of the organic acids being produced is of great importance, because they differ from each other in respect of the ability to attack structures. The attitude of a bacterial species to produce more or less lactic acid is in relation to its capacity of growth in environments with low pH values. Fermentative metabolism of the microorganism varies from homofermentative pattern to the heterofermentative one, based on the variation of the glucose concentration in the culture medium. In the presence of an excess of carbohydrates, the bacterium realizes the homofermentation (it produces lactic acid as the last final metabolite). Instead, with a limited addition of carbohydrate, the metabolism is heterofermentative with production of acetate, formate, ethanol and H₂O₂ and a small amount of lactate. Glucose digestion takes place according to the constituting cycle that consumes little energy. Glucose enters the cell via a passive transport (according to concentration gradient) but other active transport systems exist. If bacteria did not have an active transport system, they could not concentrate the glucose within the cell and would not be able to metabolize it for energy. All this will be balanced with a biofeedback system that is the repression of catabolite: the catabolite, the end product of metabolism, will be an inhibiting factor for the active transport system. The following types of microorganisms are present in the vegetable biomass for animal feed to be ensiled;
- Obligate homofermentants: they ferment the sugars producing only lactic acid; they produce, with the same speed of fermentation, a quantity of acid grater than that of heterofermentative species, and therefore they entail a significant decrease in the pH.
- Facultative heterofermentants: they ferment sugars following almost exclusively a homofermentative route. But if a shortage of carbohydrates occurs, a supply of inducible enzymes allows them to carry out the heterofermentation.
- Obligate heterofermentants: they ferment carbohydrates producing ethanol, other organic acids (lactic acid traces)

The primary action of RAPSODY is to encourage the obligate homofermentative pattern. In this way the production of lactic acid will be the highest possible. The biofeedback will manifest in a shorter time, therefore, the catabolite (lactic acid) will block the transport system active in the bacterium in shorter time.

### Key points:

Rapid reduction of oxygen present with savings of glucides useful for the next stage of fermentation. 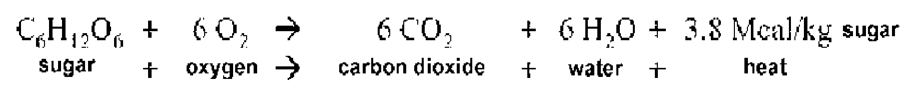 This equation indicates that the respiration of the biomass plant cells converts sugar to carbon dioxide, water and heat, with the result of a loss of dry matter and available energy. The addition of some metabolites of RAPSODY put the cells of the biomass in competition for oxygen with native microorganisms. In each gram of freshly harvested crop there are about 10¹⁰-10⁷ microorganisms. Many of these require oxygen to grow, they being strict aerobes, therefore, lead to a decrease of oxygen. Basically, this is a "natural selection" system that controls these microorganisms.

Acceleration in the reduction of pH values in the ensiled biomass by growth of hetero-fermentative native flora.

Fermentation begins when the removal of this gas starts and the bacteria capable of living both in the presence and in the absence of air (facultative aerobes) become predominant. Among these microorganisms are enterobacteria, which convert sugars in a variety of organic acids (formic, acetic, lactic and sometimes butyric acids), carbon dioxide (CO₂) and hydrogen (H₂): these acids are responsible for the early decrease of pH in the silo. This phase is referred to as latency or dormancy phase by some microbiologists: in any case, the successful silage depends very much on the outcome of this critical fermentation stage. As the fermentation proceeds, enterobacteria become less competitive, as they are particularly sensitive to the fall of the pH; their growth is inhibited when the acidity reaches a pH below 4.5, which usually occurs a few days after ensiling. However, enterobacteria tend to persist in the silage for longer periods, if the pH decreases slowly. Some metabolites are used by heterofermentative microorganisms that accelerate their development and consequently the reduction of pH. This step is reduced to about 36 hours with use of RAPSODY.

Selection of homo-fermentative lactic flora.

The fermentation of biomass is already strongly guided towards a lactic homo-fermentation by the two previous phases. The lactic homo-fermentation is strongly established when the pH reaches values close to 5.5. This is a simple fermentation in which a sugar molecule is cleaved into two molecules of lactic acid.

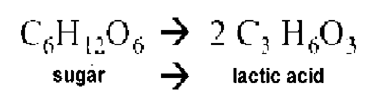

In a complex biomass however undergoing a natural fermentation, however, the achievement of a complete lactic homo-fermentation is not so easy. Hetero-fermentative lactic acid bacteria can coexist and produce acetic acid, alcohol and carbon dioxide, but these are not negative in general, if they remain under control. Homo-lactic fermentation with a strong lowering of pH starts what we might call "semi-sterilization" step of the ensiled biomass. In fact, the pH is reduced to values comprised between 3.5-4.

However, pH values lower than 5 do not guarantee the absence of contamination by microorganisms not desirable in the fermentation. These microorganisms grow in the absence of oxygen (anaerobic) and are normally present in the soil, and manure. The ability for clostridia to live without oxygen and resist pH 4.2 allows these microorganisms to compete with the lacto-bacteria even after the pH is dropped below 5. Basically, clostridia dominate the fermentation when the lactic acid bacteria do not produce lactic acid fast enough to lower the pH to a level of stability. Some species of clostridia ferment sugars and convert the lactate produced by the lacto-bacteria into butyric acid, carbon dioxide and hydrogen (see diagram below).

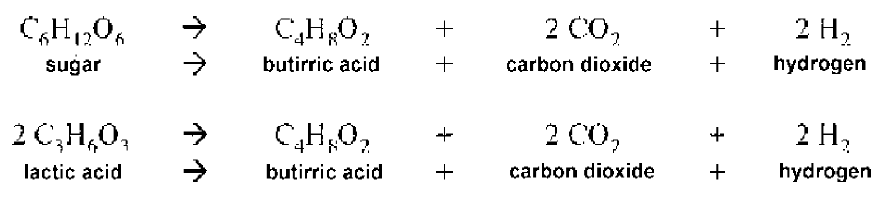

In this as in many situations in which millions of microorganisms coexist, the speed with which they the spaces or sites of attack are taken becomes fundamental. Thanks to a large and varied number of metabolites and fermentation products, RAPSODY creates an ideal habitat for homo-fermentative lacto-bacteria with particular intensity for the *L. plantarum.* The action starts from the time "0" of ensilage step, lasting until steady state of the ensiled biomass is reached. It is capable to accelerate the aerobic stability in all phases. This is not direct action, but indirect action addressed to the most important phenomena of microbial metabolism.

The development of a high number of CFU of *L. plantarum* is a very positive factor for the control of the development of negative microorganisms, clostridia and yeasts in particular, in the ensiled biomass. Recent studies have also demonstrated the *L. plantarum* ability to inhibit the growth of yeasts and clostridia in the silage plant.

Below are reported the exemplary embodiments of the present invention provided by way of illustrative examples.

### EXAMPLES

### Example 1

### Preparation of biocatalyst RAPSODY

Fermentation process: the production process is based essentially on a guided fermentation of *Sacchromyces cerevisiae* and consists of three main steps:
a- Preparation of the "Stock solution": a water-alcohol solution as a
   conditioner of the basic fermentation.
   95% ethyl alcohol 2 liters
   De-waxed propolis 450 g
   Beta carotene 4 mg
   Glycine 500 mg
   Cobalt chloride 10 mg
   Zinc chloride 200 mg
   Deionized water 2 liters
   Sodium citrate 5 mg
b- hydrolysis of the protein matrix: 5.2% protein solution of hydrolysed proteins (80% di -tri peptides).
   Soybean extraction flour 120 g
   Demineralized water 1000 ml
   Lactose-free whey flour 20 g
   NaOH based on the pH value 8.5
   Alcalase enzymes 4 mg
   Potassium sorbate 2.5 g
c- *Saccharomyces cerevisiae* multiplication phase by fermentative aerobic process of specific growth substrate: lag-phase preparing for next fermentation. The goal is the achievement of a high number of yeast cells (10⁹ CFU on the dry substance)
   Demineralized water 1000 ml
   Freeze-dried *Saccharomyces cerevisiae* yeast 150 g
   Hydrolyzate soy protein (point d) 25 g
   Sucrose 85 g
   Glucose syrup 10 ml
   Mannose 0.2 g
   Carrageenan 3 g
   Ammonium hydroxide in the amount required to achieve pH 5.2-5.4
d- fermentative phosphorylation process with the aim of biological organication of phosphates that are transformed mainly into glucose-6-phosphate, an energy unit precursor used by cells of living organisms (ATP): Phosphorylation phase. The main goal is the transformation of inorganic phosphorus in organic phosphorus in a percentage of 80% of the phosphorus.
   Sucrose 100 g
   95% ethyl alcohol 35 ml
   Glycerol 15 ml
   Mono ammonium phosphate 32 g
   Calcium chloride 220 mg
   Copper sulphate 30 mg
   Iron sulphate 25 mg
   Zinc sulphate 45 mg
   Bicalcic phosphate 5 g
   Stock solution 1 g
   picolinic acid 100 mg
   Methionine 1000 mg
   Lysine 500 mg
   Monosodium phosphate 20 g
   Protein hydrolyzate 250 ml
   Sodium sulphate 5 g
   Phosphoric acid 15 ml
e- Gradual enrichment of specific nutrients up to saturation of metabolic capacity of *Saccharomyces cerevisiae.* The nutrients used are: sulphur, calcium, phosphates, oliogelementi (iron, zinc, copper, manganese, selenium, etc.), vitamins, amino acids. This is the phase of specialization for the type of silage to be treated.
f- Enrichment of the fermentation broth with synthetic amino acids to promote the production of peptides (di- and tri- peptides) and protein structures specific of the yeast.
   Methionine 5 mg
   Lysine 10 mg
g- End of the production process by raising the temperature to 85°C (pasteurization process) to block enzymes and reproductive ability of yeast and enzymatic activity of the solution.
h- Possible drying of the solution in spray tower Dry or in a Fluid or any other system, suitable for products for food products.

In a more preferred form, RAPSODY is prepared:
fresh yeast 300 g,
soy hydrolysate 75 g,
food sugar (sucrose, glucose) 120 g,
glycerol 15 g,
mono ammonium phosphate 30 g,
bicalcic orthophosphate 7 g,
monosodium phosphate 22 g,
sodium sulphate 6 g,
lysine 1 g,
methionine 2 g,
zinc chloride 12 g,
copper sulphate 9 g,
iron sulphate 9 g,
manganese sulphate 4 g.

Special ingredients are added to the mixture: bees propolis with natural preservative function and vitamins as specified in paragraph relating to the preparation of the "Stock solution".

Formulations can be prepared with, for example, reduced presence of microelements, in favor of the sugar part and the phosphates. Variations are in the order of 10-20% maximum with respect to sugars and phosphates, to the detriment of the presence of mineral micro-elements.

If the balance is shifted towards the oligo-elements for targeted formulations concentrations of about 90-100 grams of the micro-elements can be obtained. The same applies for the Lysine and Methionine amino acids when the balance is shifted towards the protein amount.

In protein formulas a concentration of 12 g per liter of solution can be reached as the sum of the two main amino acids.

### Example 2

### Use of the biocatalyst

Due to its ability to stimulate the native lactic flora of the biomass with targeted formulations, the biocatalyst can be used with any type of plant silage.

In particular:
a- Endiled cereals (corn, barley, triticale, oats, rye, sorghum) both as full plant and simply as portions of the plants. For example: wet grain, whole ear, aerial parts of the plant.
b- Ensiled forage grasses (alfalfa, ryegrass, permanent pastures), preferably pre-dried with humidity between 30-60%.
c- Ensiled flour of seeds of oleaginous crops after oil pressing (extraction flour of: soybean, rapeseed, sunflower) at the semi-solid state with humidity of biomass to ensile comprised between 20 and 40%.
d- silage of wheat milling by-products (bran, groats, middlings)
e- Silage of a mixture comprising fractions of products listed in points C and D. The additive inclusion level varies according to humidity and the vegetative biomass condition. In silage of fodder plants (A and B) the inclusion level varies from 0.5 to 3 kg per ton of biomass. Un the ensilage of products at the semi-solid form (C and D) the inclusion level varies from 0.5 to 5 kg per ton of wet biomass.

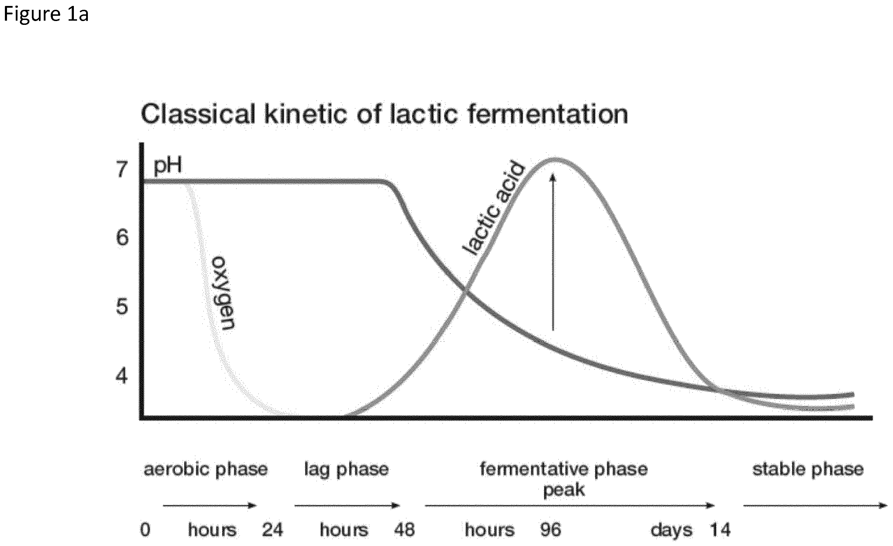

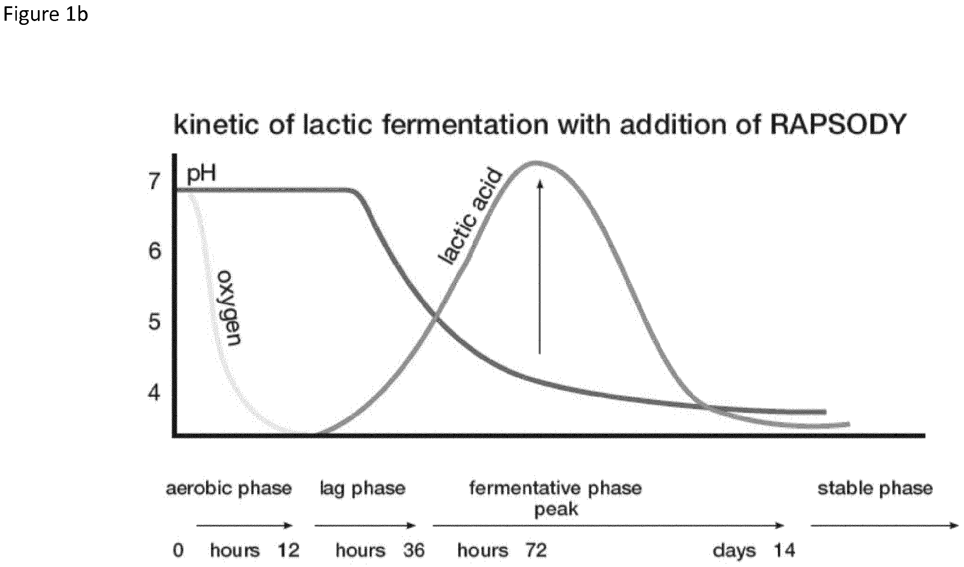

As shown in Figure 1, the use of the biocatalyst according to the invention allows an acceleration of the initial phase of fermentation. Depending on the type of final product, the fermentation can be stopped at different times to take advantage of certain nutritional values of the biomass for specific purposes.

Figure 1a, shows a graph plotting the trend of a classical fermentation of a plant biomass ensiled for livestock use without the addition of a biocatalyst, while Figure 1b shows the same type of graph with the addition of the biocatalyst according to the invention.

From the detailed description and from the Examples given above, the advantages achieved by the process and the product of the present invention are evident. In particular, the biocatalyst has surprisingly and advantageously proven to be suitable as an additive of silage foods, stimulating the fermentation ability of native lactobacilli of plant biomass.

## Claims

1. A process for the preparation of a biocatalyst comprising the steps of:
a. mixing an amount of flour hydrolyzate with one or more sugars, minerals, aminoacids, propolis and vitamins thereby producing a dry mixture;
b. adding water to the dry mixture of step a. in order to obtain a suspension;
c. fermenting the suspension of step b. with yeast belonging to the Saccharomyces cerevisiae species for about 10-14 hours at a temperature in the range of 25°C to 35°C, in order to obtain a fermentation broth;
and
d. pasteurizing the fermentation broth of step c. at a temperature in the range of 85°C to 90°C for about 15-20 minutes.

2. The process according to claim 1, wherein the flour hydrolyzate preferably is a flour hydrolyzate of soy, peas, rape, sunflowers, yeast auto lysate, wheat by-products, potato protein concentrate or a mixture thereof.

3. The process according to anyone of claims 1 or 2, wherein the sugar is selected from the group consisting of dextrose, glycerol, mannose, sucrose, fructose or a mixture thereof.

4. The process according to anyone of claims 1 to 3, wherein minerals are chosen from the group consisting of phosphates, sulphates, mineral microelements, preferably mono-ammonic phosphate, bicalcic orthophosphate, monosodium phosphate, sodium sulphate, zinc chloride, zinc sulphate, zinc oxide, copper sulphate, copper chloride, iron sulphate, iron chloride, manganese sulphate, sulphur flower or a mixture thereof.

5. The process according to anyone of claims 1 to 4, wherein the amino acids are chosen from the group consisting of lysine, methionine, threonine, tryptophan, valine, cysteine, arginine or a mixture thereof.

6. The process according to anyone of claims 1 to 5, wherein:
the dry mixture of step a. comprises soy hydrolysate, food sugar, (sucrose, glucose), glicerol, mono-ammonic phosphate, bicalcic orthophosphate, monosodium phosphate, sodium sulphate, lysine, methionine, zinc sulphate, zinc oxide, copper sulphate, copper chloride, iron sulphate, iron chloride, manganese sulphate, sulphur flower or a mixture thereof.

7. Biocatalyst obtainable from the process according to anyone of claims 1 to 6.

8. Use of the biocatalyst according to claim 7, as an additive for animal and livestock food.

9. The use according to claim 8, wherein said animal and livestock food is a silage.

10. The use according to anyone of claims 7 or 8, wherein said animal and livestock food is in a liquid form or presents a water percentage from 15% to 80%.
